# EUROPEAN PATENT APPLICATION

(11) **EP 4 714 359 A1**
(43) Date of publication of application: **25.03.2026**
(21) Application number: 24200982.7
(22) Date of filing: 18.09.2024
(51) Int. Cl.: A61B 6/10, G21F 3/025

(54) **PROTECTION AND COMMUNICATION DURING MEDICAL X-RAY INTERVENTIONS**

(71) Applicant: Koninklijke Philips N.V., 5656 AG Eindhoven (NL)
(72) Inventor: VAN PELT, Roy Franciscus Petrus, Eindhoven (NL); ELENBAAS, Thijs, Eindhoven (NL); HENDRIKS, Bernardus Hendrikus Wilhelmus, 5656AG Eindhoven (NL)
(74) Representative: Philips Intellectual Property & Standards

(57) **Abstract**

The present invention relates to providing communication during an operation or other type of medical intervention. In order to provide an improved communication tool with a facilitated setup, a protective communication system (10) for medical X-ray interventions comprises a radiation protection arrangement (12) with at least one X-ray radiation shielding (14) configured to protect at least a part of the user from X-ray radiation and a wearable holding structure (16) configured for a temporal attachment to a user during operation of X-ray radiating equipment. The protective communication system also comprises a communication arrangement (22) with a microphone (24), an acoustic output device (26) and an energy supply device (28) with at least one battery. The at least one X-ray radiation shielding is attached to the wearable holding structure. The energy supply device is supported by the radiation protection arrangement.

## Description

### FIELD OF THE INVENTION

The present invention relates to providing communication during an operation or other type of medical intervention. The present invention relates in particular to a protective communication system for medical X-ray interventions, to a medical X-ray intervention setup and to a method for providing communication during medical X-ray interventions.

### BACKGROUND OF THE INVENTION

In an operation room providing e.g. an interventional X-ray setting, there is continuous communication, for example among staff and also between the examination room and the control room. For this purpose, there is a pressing need to have a proper acoustic communication system between these areas. For this purpose, many solution directions have been explored in the past, including various (noise-cancelling) headsets and directional microphones in the examination room. However, it has been shown that these may be cumbersome to wear or complex in their integration into the increasingly equipped operation room. Further, some of the solutions have proven to be insufficient, either because they are discarded due to the discomfort of wearing a device, or because of a lack of good audio quality when picked up in a busy intervention room.

### SUMMARY OF THE INVENTION

There may thus be a need for an improved communication tool with a facilitated setup.

The object of the present invention is solved by the subject-matter of the independent claims; further embodiments are incorporated in the dependent claims. It should be noted that the following described aspects of the invention apply also for the protective communication system for medical X-ray interventions, for the medical X-ray intervention setup and for the method for providing communication during medical X-ray interventions.

According to the present invention, a protective communication system for medical X-ray interventions is provided. The system comprises a radiation protection arrangement with at least one X-ray radiation shielding configured to protect at least a part of the wearable holding structure. Further, the energy supply device is supported by the radiation protection arrangement.

As an advantage, the weight of the energy supply device is carried by the radiation protection arrangement, which is usually quite heavy itself due to the material needed for the radiation absorption. Hence, the user experiences the additional weight of the communication arrangement rather less.

According to an example, the radiation shielding comprises a user-worn protection clothing accessory of at least one of the group of thyroid protection shield, lead glasses, lead apron, lead cap and lead gloves.

All these have in common that they are usually showing some weight due to their protective function; carrying the battery, or also additional components of the audio system, result in a relative small increase of weight.

According to an example, a control circuit for operating the microphone and the acoustic output device is provided. In an option, the control circuit is supported by the radiation protection arrangement.

According to an example, the microphone is provided integrated into the radiation shielding. In an option, the acoustic output device is provided integrated into the radiation shielding.

As an effect, a comfortable solution with improved audio quality is provided. The solution provided herewith brings the audio record close to the physician, which enables better audio quality, and does not add discomfort in wearing, as it is integrated into necessary radiation shielding.

As an advantage, a clear communication is provided despite background noises, and the communication is sufficiently discrete in use, which is of particular advantage for interventions in which patients remain awake.

Embedding a microphone as part of the radiation shielding brings the recording close to the physician which improves the audio quality. Combined with noise-cancellation technology will further improve the audio signal as received in the control room.

According to an example, a head worn device is provided, and in-ear or bone-conducting speakers are carried by the head worn device. In an option, the head worn device is provided as X-ray shielding glasses.

According to an example, the radiation shielding comprises a point of control for user interaction. In an option, audio controls are provided on the radiation protection arrangement. In an option, a touch control module interface is provided on the radiation shielding.

According to an example, the radiation shielding comprises a camera system that provides contextual information. The camera system is powered by a battery in the radiation protection arrangement. The camera system is connected to a wireless communication.

According to an example, the battery is provided as a flat and flexible structure.

According to the present invention, also a medical X-ray intervention setup is provided. The setup comprises an X-ray imaging system with an X-ray source and, as an option, an X-ray detector, and at least one protective communication system according to one of the preceding examples. During operation of the X-ray imaging system, at least one user is wearing one of the at least one protective communication systems.

According to the present invention, also a method for providing communication during medical X-ray interventions is provided. The method comprises the following steps: providing at least one protective communication system according to one of the preceding examples; wearing of the at least one protective communication system by at least one user; and communicating of the at least one user with another staff member via the communication arrangement.

According to an aspect, better audio quality for the physician is provided by bringing the microphone close employing a protection measure used anyway, such as a radiating shielding. To embed the microphone in the overall interventional system, transceiver units are required in the radiation shielding as well as the system to establish two-way communication. In an option, a speaker/headphone integration is provided. In another option, user interaction provides controlling the audio.

For the implementation, electronics and controls are embedded in the shield, maintaining a smooth surface to ensure sterility and good cleanability.

As a result, less 'out-loud' communication going back and forth between the exam and control room is provided, preventing the potential need to break sterility. The intercom functionality is improved which leads to an improved experience of the whole interventional or examination system.

According to an aspect, already existing X-ray shielding devices like lead glasses in combination with the radiation shielding are re-used and modified to enable the communication link with the exam room. The glasses will offer speakers close to the ear or through bone conduction, and in that way minimally distract bystanders. The design can be kept small and lightweight because the battery and control board will reside in the radiation shielding. Thus, additional weight and ergonomic burden will be minimal as compared to regular lead glasses.

The present solution provides improved audio quality without discomfort for the physician, with the physician in control of adjusting the audio setting when necessary. Furthermore, the solution allows for additional tailored information feedback to each person in the interventional room without the introduction of additional discomfort. A longstanding clinical problem and associated technical challenges are thus addressed.

As a further advantage, the present solution invention is independent of the clinical application in the interventional domain, e.g. neuro interventions, cardio related interventions, vascular interventions, oncology or others.

Contrary to headsets that are a significant piece of hardware to wear, or ear plugs that block the ear, the present solution provided minimum additional constraints for the user. Further, also an extended battery time is provided. The data processing circuitry can be provided integrated into the radiation shielding. As an option, also the energy storage function is integrated into the radiation shielding.

Apart from improved communication between the staff members themselves, or between the physician/interventionalist and the control room, or staff within the examination room with others outside the examination room, the tailored communication to each of the personnel also enables additional feedback on important information. For example, for radiation safety, the stray radiation dose measured by for instance badges can be directly communicated to the person wearing these badges. Furthermore, when such a personnel communication link exists, also more context information can be provided to the control room. For instance, when the physician in the treatment room is wearing a camera, the personnel in the control room can look over the shoulder of the physician and can determine better when to disturb the physician and when not.

According to an aspect, a solution is provided that brings audio recording, playback, and control into combination with the radiation shielding. In an option, X-ray shielding glasses used by interventional physicians in the exam room are part of the arrangement as well.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a protective communication system for medical X-ray interventions.
Fig. 2 shows the example of the protective communication system for medical X-ray interventions of Fig. 1 with further options.
Fig. 3 schematically shows further examples of the protective communication system for medical X-ray interventions in the context of an example of a medical X-ray intervention setup.
Fig. 4 shows basic steps of an example of a method for providing communication during medical X-ray interventions.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

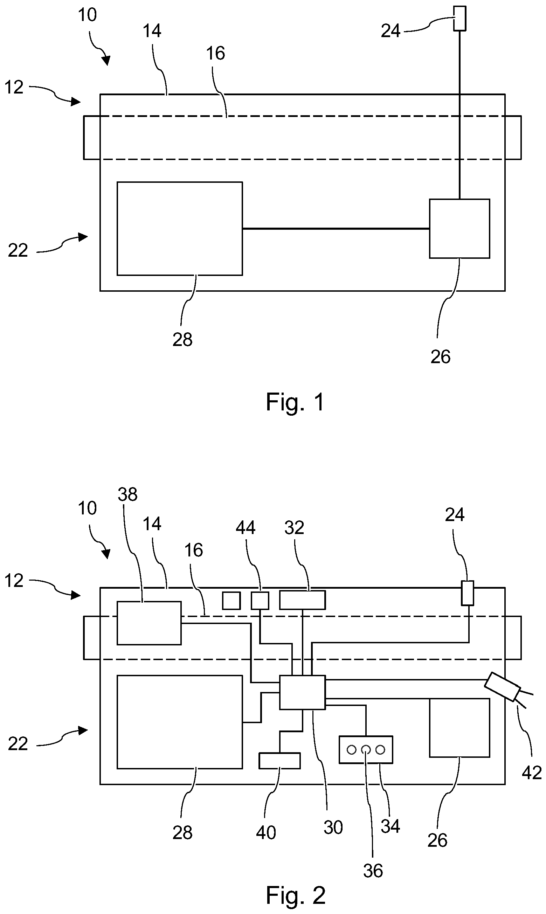

Fig. 1 schematically shows an example of a protective communication system 10 for medical X-ray interventions. The protective communication system 10 comprises a radiation protection arrangement 12 with at least one X-ray radiation shielding 14 configured to protect at least a part of the user from X-ray radiation, and a wearable holding structure 16 configured for a temporal attachment to a user during operation of X-ray radiating equipment. The protective communication system 10 also comprises a communication arrangement 22 with a microphone 24, an acoustic output device 26 and an energy supply device 28 with at least one battery. The at least one X-ray radiation shielding 14 is attached to the wearable holding structure 16 and the energy supply device 28 is supported by the radiation protection arrangement 12.

In Fig. 1, connecting lines indicate a functional connection between the energy supply device 28, the acoustic output device 26 and the microphone 24. The functional connection comprises data connection and/or energy transmission. The connection can be wireless, e.g. for data connecting, or wire-bound, for example for energy transfer. However, the actual connection cables are not further shown in the drawings. Similar applies for the options shown in Fig. 2.

The term "protective communication system" relates to a bifunctional wear-on device providing communication and protection for a user during X-ray radiation procedures. The protective communication system can also be referred to as a communication system that also provides X-ray protection for a user. The present invention provides a communication system integrated with a wear-on radiation protection. The protective communication system can also be referred to as a protection communication system, a protecting communication system, a protecting communicating system, a communication protection system, a communicative protection system, a communicating protection system or a communicating protecting system

The term "radiation protection arrangement" relates to radiation protective means or shieldings and the respective holding structure like straps, belts and the like, to temporarily wear or carry the radiation protection arrangement by the user.

The term "radiation shielding" relates to the shielding material that provides the radiation absorption.

The term "holding structure" relates to the gear provided to wear the radiation protection arrangement. The radiation shielding is thus connected to the holding structure and the holding structure is applied by the user. In an option, the radiation shielding, and the holding structure are separate parts connected to each other, either permanently or in a detachable manner. In another option, the radiation shielding, and the holding structure are integrated with each other.

The term "communication arrangement" relates to the means providing communication between the user and other staff members. Basically, the communication arrangement provides a microphone, for recording the user's voice and transmitting the respective signals to other staff members, and a loudspeaker or other form of providing acoustic signals to the user, for example for transmitting the voice communication by other staff members or other forms of audible signals like warning signals, trigger signals, steering signals or other computer generated signals provided in form of acoustic signals that the user wearing the protective communication system can hear with her/his ears.

The communication arrangement can also be referred to as an audio system.

The term "microphone" relates to a device capable of receiving acoustic waves like voices and other sound sources and that generate a signal for transmitting further.

The term "acoustic output device" relates to a device capable of receiving a signal and generating a respective sound impression or experience by the user. As an example, sound waves can be generated that the user can hear. For example, the acoustic output device is a loudspeaker, which can be arranged close to the user's ear or as in-ear speaker. As another example, vibrations are generated and transmitted to the user's skull, i.e. the user's bone head structure, such that the user experiences the sound as if it would be provided as sound waves. For example, the acoustic output device is a vibrating element, which can be arranged on the skull close to the user's ear.

The term "energy supply device" relates to a source of electric energy to operate the microphone and the acoustic output device. The energy supply device is an independent source, e.g. with no further cable connection to an external source. As an example, the energy supply device is a rechargeable battery. As another example, the energy supply device is a disposable battery.

The term "supported by" relates to holding the energy supply device, i.e. carrying the weight of the energy supply device by the radiation protection arrangement. As an example, the energy supply device is mounted or fixed to the radiation shielding. As another example, the energy supply device is mounted or fixed to the holding structure.

In a first example, the energy supply device 28 is supported by the radiation shielding 14. In a second example, the energy supply device 28 is supported by the holding structure 16.

In an option of the example of Fig. 1, the radiation shielding 14 comprises a user-worn protection clothing accessory of at least one of the group of a thyroid protection shield, lead glasses, a lead apron, a lead cap and lead gloves.

The term thyroid protection shield relates to a radiation protection shielding worn by the user around her/his neck. The shield comprises an inlet that at least partly absorbs X-ray protection. The shield is usually somewhat flexible to allow a comfortable fit around the neck. The thyroid protection shield can also be referred to as thyroid shield or neck protection shield.

The term lead glasses relates to glasses with glass inserts that at least partly absorb X-ray protection. The glass inserts are usually provided as a transparent base material containing particles of lead or other useable substances to provide absorption of X-ray radiation.

The term lead apron relates to a radiation protection shielding worn by the user in front of or partly around her/his main corp. The lead apron can also be referred to as lead apron shielding.

The term lead cap relates to a radiation protection shielding worn by the user on her/his head. The lead cap can also be referred to as lead cap shield.

In an option, the communication arrangement is partly integrated into the user-worn protection clothing accessory.

In another option, the communication arrangement is completely integrated into the user-worn protection clothing accessory. In a variation, the communication arrangement is integrated into one of the user-worn protection clothing accessories. In another variation, two or more of the user-worn protection clothing accessories are provided and the communication arrangement is integrated into two or more of the user-worn protection clothing accessories.

As an example, the protection arrangement comprises a thyroid protection shield and protection glasses. The audio feedback device is integrated into the protection glasses, whereas the energy device like a battery is integrated into the thyroid protection shield.

Fig. 2 shows the example of the protective communication system for medical X-ray interventions of Fig. 1 with further options.

In an option of the example of Fig. 2, a control circuit 30 for operating the microphone 24 and the acoustic output device 26 is provided. As an option, the control circuit 30 is supported by the radiation protection arrangement 12.

In a first example, the control circuit 30 is supported by the radiation shielding 14. In a second example, the control circuit 30 is supported by the holding structure 16.

The term "control circuit" relates to electric circuitry provided for operating the communication arrangement.

The protection shield 14 brings the microphone and speaker close to the physician, eliminating background interference. Due to its inherent weight, the combination will not suffer from the addition of a battery.

In an option of the example of Fig. 2, the microphone 24 is provided integrated into the radiation shielding 14. As an option, in addition or alternatively, the acoustic output device 26 is provided integrated into the radiation shielding 14.

In an option, the glasses will offer speakers close to the ear or through bone conduction, and in that way minimally distract bystanders. The speakers will be powered through the battery pack integrated into the radiation shielding and thus stay lightweight as much as lead glasses can be. The combination of both allows for a unique combination of i) re-using already present accessories, ii) introducing minimal interference for the user or others and iii) does not degrade ergonomics.

In an option of the example of Fig. 2, a wireless audio transceiver 32 is provided integrated into the radiation shielding 14.

The term "wireless audio transceiver" relates to an interface that allows to receive and to send signals representing audio information like the recorded sound of the user's voice or some other staff member's voice.

In an option, further audio infrastructure is arranged integrated into the radiation shielding.

As an example, the audio of the physician's voice is connected to an existing audio system, e.g., connect to the interventional X-ray intercom system. This requires submitting the audio stream of the physicians' voices and receiving return communication audio from the control room staff. A wireless audio transceiver solution is provided embedded in the radiation shielding as well as a connecting component as part of the interventional X-ray (intercom) system. These two modules connect to ascertain two-way communication. Different means for wireless communication are conceivable, such as a DECT, Bluetooth protocol or ad-hoc Wi-Fi connection.

In an option, shown in Fig. 2, the radiation shielding comprises a point of control 34 for user interaction. As an option, audio controls 36 are provided on the radiation protection arrangement. As another option, in addition or alternatively, a touch control module 38 interface is provided on the radiation shielding 14.

The term "audio controls" relates to a user interface for controlling audio functions like volume, tone or balance of the generated sound signals.

The term "touch control module" relates to an interface that is touch sensitive for user interaction in order to provide control of various functions, like the audio control functions or other control of equipment provided in the vicinity and handled by the user and/or the staff members.

As an example, the radiation shielding provides a user interface for functions like mute/unmute and volume control. Advantageously, the user interface is arranged within the user's sterile zone.

In another option, the user interface e.g. the user controls, on the radiation shielding are connected to an interventional touchscreen module (TSM), as an alternative point to control the audio or other functions.

The touch control module can also be referred as touch sensitive module, TSM.

Having electronics integrated into the radiation shielding also allows for (touch) buttons to be integrated to allow easy access to control the audio, such as mute/unmute, volume, and noise cancellation levels. The buttons are easily accessible in a comfortable position and always with the physician or other user wearing the protective communication system.

With the warm integration between the transceiver in the radiation shielding and the interventional system, the touch control module allows for an additional user interface to control the audio.

In an option, shown in Fig. 2, a data exchange interface 40 is provided to provide access to the interventional intercom system.

The term "data exchange interface" relates to an interface that allows to receive and to transmit data in the format as used by the intercom system in the medical operation room.

In an option, shown in Fig. 2, the radiation shielding comprises a camera system 42 that provides contextual information. The camera system 42 is powered by a battery in the radiation protection arrangement 12, e.g. the at least one X-ray radiation shielding 14. The camera system 42 is connected to a wireless communication. In an example, the camera system 42 comprises one camera; in another example, two or more cameras are provided.

The term "camera system" relates to a system providing optical recording like the visible light spectrum or also infrared light spectrum or ultraviolet light spectrum. The camera system generates and transfers respective signals for further image processing or visual presentation.

In a further option, the protection arrangement, e.g. in the radiation shielding of in the protection glasses, comprises a camera that allows "to look over the shoulders of the physician" to provide more context to the situation in the treatment room. For instance, this can be useful to know when to interrupt or not the physician. Instead of a camera, also a position sensor can be used to provide context to the examination room.

In an option, shown in Fig. 2, the radiation shielding 14 comprises additional sensors 44 such as radiation dose sensors and the measured data is directly communicated to the physician.

The term "additional sensors" relates to sensors other than the acoustic sensor in form of the microphone. The sensors provide additional data and parameter.

In an option, additional sensors and feedback is provided in a tailored way to the personnel in the treatment room. For instance, information from a radiation dose badge, such as DoseAware, can be directed be presented to the physician in audio feedback or mechanical feedback like a vibration device incorporated in the protection arrangement, e.g. in the radiation shielding of in the protection glasses.

In an option, not shown in detail, the radiation shielding comprises tactile feedback means.

The term "tactile feedback means" relates to providing feedback that the user experiences via her/his tactile senses. The feedback can be provided to various parts of the body, like the main corps or extremities like the arms or legs or even the neck or head portions.

In an option, the tactile feedback means are provided as mechanical means, e.g. providing vibration feedback.

In another option, the tactile feedback means are provided as electronic means, e.g. providing feedback by small electric currents.

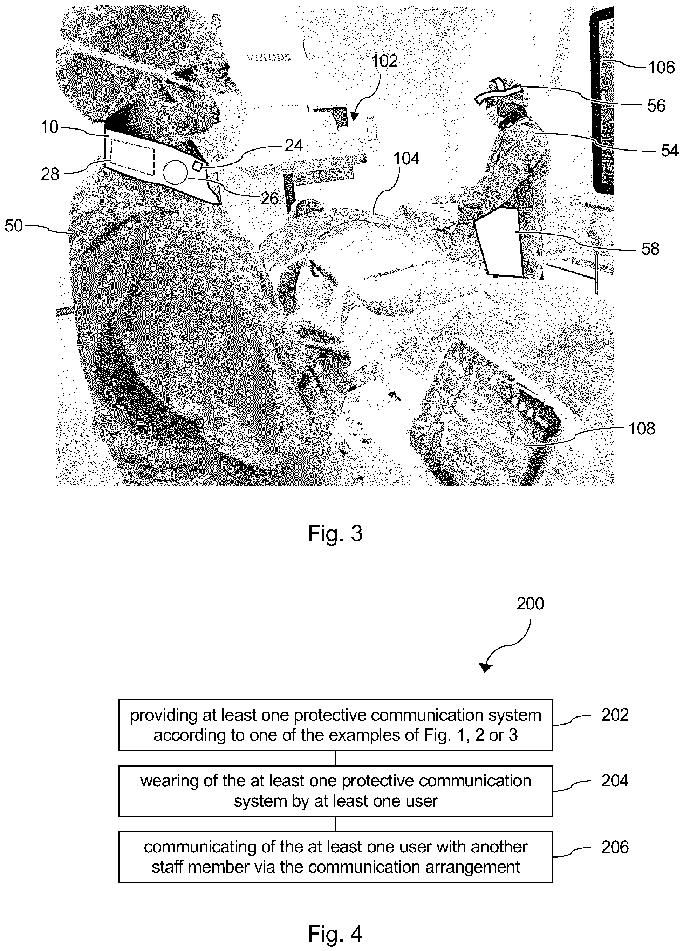

In an option, indicated in Fig. 2 and Fig. 3, the at least one battery, i.e. the energy supply device 28, is provided as a flat and flexible structure.

In an option, the battery is provided as an additional shielding layer integrated in the radiation shielding.

It is noted that the various options are provided in various combinations, e.g. all combined as illustrated, e.g. control circuit 30, wireless audio transceiver 32, point of control 34 for user interaction, audio controls 36, touch control module 38, data exchange interface 40, camera system 42, additional sensors 44 and tactile feedback means. But also just in selected combinations such as, for example: a combination of control circuit 30, wireless audio transceiver 32, point of control 34 for user interaction, audio controls 36, touch control module 38, data exchange interface 40, camera system 42, additional sensors 44; or a combination of control circuit 30, wireless audio transceiver 32, point of control 34 for user interaction, audio controls 36, touch control module 38, data exchange interface 40, camera system 42; or a combination of control circuit 30, wireless audio transceiver 32, audio controls 36, touch control module 38, data exchange interface 40, camera system 42; or a combination of control circuit 30, wireless audio transceiver 32, point of control 34 for user interaction, audio controls 36, touch control module 38, data exchange interface 40, camera system 42, additional sensors 44 and tactile feedback means; or a combination of wireless audio transceiver 32, point of control 34 for user interaction, audio controls 36, data exchange interface 40, camera system 42, additional sensors 44; or a combination of control circuit 30, point of control 34 for user interaction, data exchange interface 40, camera system 42; or a combination of control circuit 30, wireless audio transceiver 32, touch control module 38, data exchange interface 40; or a combination of control circuit 30, wireless audio transceiver 32, touch control module 38, data exchange interface 40; or a combination of wireless audio transceiver 32, point of control 34 for user interaction, audio controls 36, touch control module 38, data exchange interface 40; or a combination of control circuit 30, wireless audio transceiver 32, point of control 34 for user interaction, audio controls 36, touch control module 38, data exchange interface 40; or any other technically useful combination thereof.

In Fig. 3, a user 50 is shown wearing a shielding 52 around the neck region as an example of the protective communication system 10.

In an option, shown in Fig. 3 for another user 54 in the background, a head worn device 56 is provided as another example of the protective communication system 10. As an option, in-ear or bone-conducting speakers are carried by the head worn device 56. As an option, the head worn device is provided as X-ray shielding glasses (not shown in detail).

As another example of the protective communication system 10, an apron 58 is indicated.

The term "head worn device" relates to any kind of device worn by the user on her/his head. The head worn device can be provided in the manner of spectacles or a headband.

As an example, X-ray shielding lead glasses are provided. In an option, the design is small and lightweight because the battery and control board reside in the radiation shielding. Thus, additional weight and ergonomic burden will be minimal as compared to regular lead glasses.

Fig. 3 schematically shows further examples of the protective communication system 10 for medical X-ray interventions in the context of an example of a medical X-ray intervention setup 100. The medical X-ray intervention setup 100 comprises an X-ray imaging system 102 with an X-ray source and an X-ray detector. The medical X-ray intervention setup 100 further comprises at least one example of the protective communication system 10 according to one of the examples above. During operation of the X-ray imaging system, at least one user is wearing one of the at least one protective communication systems.

In Fig. 3, a subject 104 is indicated on a subject support like an operation table. Further, a display 106 is indicated in the background and a control console 108 in the foreground.

In an option, not shown in detail, the communication setup comprises a personal link to the personnel. The personal link can be used to provide feedback on devices and equipment directly to dedicated persons only.

The setup can also be referred to as medical X-ray intervention arrangement or as medical X-ray intervention scenario.

Fig. 4 shows basic steps of an example of a method 200 for providing communication during medical X-ray interventions. The method comprises the following steps:
- In a first step 202, at least one protective communication system according to one of the examples above is provided.
- In a second step 204, at least one protective communication system is worn by at least one user.
- In a third step 206, the at least one user is communicating with another staff member via the communication arrangement provided by the protective communication system.

The term "subject" may also be referred to as individual. The "subject" may further also be referred to as patient, although it is noted that this term does not indicate whether any illness or disease is actually present with the subject.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A protective communication system (10) for medical X-ray interventions, the system comprising:
- a radiation protection arrangement (12) with:
- at least one X-ray radiation shielding (14) configured to protect at least a part of the user from X-ray radiation, and
- a wearable holding structure (16) configured for a temporal attachment to a user during operation of X-ray radiating equipment; and
- a communication arrangement (22) with:
- a microphone (24);
- an acoustic output device (26); and
- an energy supply device (28) with at least one battery;
wherein the at least one X-ray radiation shielding is attached to the wearable holding structure; and
wherein the energy supply device is supported by the radiation protection arrangement.

2. System according to claim 1, wherein the radiation shielding comprises a user-worn protection clothing accessory of at least one of the group of:
- thyroid protection shield;
- lead glasses;
- lead apron;
- lead cap; and
- lead gloves.

3. System according to claim 1 or 2, wherein a control circuit (30) for operating the microphone and the acoustic output device is provided; and
wherein the control circuit is supported by the radiation protection arrangement.

4. System according to claim 1, 2 or 3, wherein the microphone is provided integrated into the radiation shielding; and
wherein the acoustic output device is provided integrated into the radiation shielding.

5. System according to one of the preceding claims, wherein a wireless audio transceiver (32) is provided integrated into the radiation shielding.

6. System according to one of the preceding claims, wherein a head worn device (56) is provided, and wherein in-ear or bone-conducting speakers are carried by the head worn device; and
wherein the head worn device is provided as X-ray shielding glasses.

7. System according to one of the preceding claims, wherein the radiation shielding comprises a point of control (34) for user interaction;
wherein audio controls (36) are provided on the radiation protection arrangement; and
wherein a touch control module (38) interface is provided on the radiation shielding.

8. System according to one of the preceding claims, wherein a data exchange interface (40) is provided to provide access to the interventional intercom system.

9. System according to one of the preceding claims, wherein the radiation shielding comprises a camera system (42) that provides contextual information;
wherein the camera is powered by a battery in the protection shield; and
wherein the camera is connected to a wireless communication.

10. System according to one of the preceding claims, wherein the radiation shielding comprises additional sensors (44) such as radiation dose sensors and the measured data is directly communicated to the physician.

11. System according to one of the preceding claims, wherein the radiation shielding comprises tactile feedback means.

12. System according to one of the preceding claims, wherein the battery is provided as a flat and flexible structure.

13. A medical X-ray intervention setup (100), comprising:
- an X-ray imaging system (102) with at least an X-ray source; and
- at least one protective communication system (10) according to one of claims 1-12;
wherein, during operation of the X-ray imaging system, at least one user is wearing one of the at least one protective communication systems.

14. A method (200) for providing communication during medical X-ray interventions, comprising the following steps:
- providing (202) at least one protective communication system according to one of the claims 1-12;
- wearing (204) of the at least one protective communication system by at least one user; and
- communicating (206) of the at least one user with another staff member via the communication arrangement.
